# EUROPÄISCHE PATENTSCHRIFT

(11) **EP 1 595 942 B1**
(45) Veröffentlichungstag und Bekanntmachung des Hinweises auf die Patenterteilung: **05.03.2008**
(21) Anmeldenummer: 05008863.2
(22) Anmeldetag: 22.04.2005
(51) Int. Cl.: C12N 1/04, C12N 11/04, B01J 2/16

(54) **Verfahren zur Verkapselung bzw. Immobilisierung von Mikroorganismen**
Process for encapsulating or immobilisation of microorganisms
Procédé d'enrobage ou immobilisation de microorganismes

(30) Priorität: 11.05.2004 DE 102004023725
(43) Veröffentlichungstag der Anmeldung: 16.11.2005
(73) Patentinhaber: Glatt Ingenieurtechnik GmbH, D-99427 Weimar (DE)
(72) Erfinder: Rümpler, Karlheinz, Dr., 99425 Weimar (DE); Jacob, Michael, 99427 Weimar (DE); Meiners, Jean-Antoine, 2036 Cormondrèche (CH)
(74) Vertreter: Maucher, Wolfgang

(56) Entgegenhaltungen:
- EP-A- 1 407 814
- WO-A-01/68808
- WO-A-20/04108911
- DE-A1- 10 035 556

## Beschreibung

Die Erfindung betrifft ein Verfahren zur Herstellung von Partikeln mit den im Oberbegriff des Patentanspruches 1 genannten Merkmalen.

Die Haltbarmachung von lebensfähigen Mikroorganismen über längere Zeiträume ohne Verminderung der Überlebensrate gestaltet sich im allgemeinen schwierig. Insbesondere wechselnde Umgebungsbedingungen führen zu einer Reduzierung der Lebensfähigkeit der Bakterien. Weiterhin besteht das Ziel, Mikroorganismen gegen thermische und mechanische Einflüsse, die Wirkung von Magensaft oder ähnliche Einwirkungen zu schützen.

Es ist allgemein bekannt, dass Mikroorganismen, die unter trockenen Bedingungen konserviert werden, in eine Art Ruhezustand gelangen und dann über eine längere Zeitdauer lagerfähig sind. Die Trocknung der Kulturen erfolgt bisher unter Verwendung bekannter Trocknungstechnologie. Um die thermische Belastung gering zu halten, werden bisher Gefriertrocknungs- oder Vakuumtrocknungsprozesse genutzt. Dadurch kann das Temperaturniveau relativ niedrig gehalten werden, und die Mikroorganismen behalten einen relativ großen Teil ihrer Lebensfähigkeit. Die mit diesen Verfahren hergestellten Trockenstoffe sind nicht gut rieselfähig und haben keine enge Korngrößenverteilung. Außerdem sind die Trocknungsverfahren technisch aufwendig bzw. benötigen sehr viel Trocknungszeit.

Es ist Stand der Technik, dass es verschiedene Verfahren zur Behandlung und Konservierung von Mikroorganismen gibt. Diese werden nachfolgend kurz erläutert:

In der US-PS 4046921 wird ein Verfahren zur Kultivierung von Mikroorganismen in einem Wirbelschichtapparat beschrieben. Dabei wird eine Kultur in fester Form durch ein Gas fluidisiert und eine Flüssigkeit zerstäubt eingebracht. Die Apparatur wird zunächst mit einer Startfüllung beladen. Anschließend wird eine Flüssigkeit auf das aus der Kultur bestehende Wirbelbett gesprüht, um den Wassergehalt der Kultur im Bereich von 15-70% (v/w) bzw. vorzugsweise 25-60% (v/w) einzustellen. Die Flüssigkeit kann im einfachsten Fall Wasser sein oder auch Nährmittel, anorganische Salze, Pilze, Bakterien enthalten. Der Apparat wird verwendet, um das Wachstum von Mikroorganismen, die Produktion von Enzymen usw. zu kontrollieren.

Aus der US-PS 4888171 ist ein granuliertes Produkt mit schichtweiser Struktur bekannt, welches die Lagerfähigkeit verbessert und das dazugehörende Herstellungsverfahren. Als Basismaterial werden getrocknete Zellen von Mikroorganismen verwendet. Ziel ist es, das Granulat, welches getrocknete Mikroorganismus-Zellen enthält, gegen eindringende Feuchte und Luftsauerstoff zu schützen und eine hohe Lagerstabilität bei guter Lebensfähigkeit zu erreichen. Es wird beschrieben, dass auf einen fluidisierten Trägerstoff ein geschmolzenes wasserfreies Bindermaterial aufgesprüht wird. Gleichzeitig werden getrocknete Zellen von Mikroorganismen in das Wirbelbett dosiert, die sich dann auf dem Trägermaterial unter Wirkung des Bindemittels als eine Art Hüllschicht anlagern.

In der EP-A- 1265983 wird die Herstellung von Partikel beschrieben, die getrocknete lebende Mikroorganismen enthalten. Die Partikel werden derart hergestellt, dass eine geschmolzene hydrophobe Substanz in eine Masse aus getrockneten Mikroorganismen eingedüst wird. Die getrockneten Mikroorganismen werden in einer Prozesskammer durch einen temperierten Gasstrom und eine rotierende Bodenplatte umgewälzt.

Aus der JP 62277196 kennt man bereits einen Apparat, der verschiedene Arten von Trägerstoffen enthält. Diese Träger werden mit Mikroorganismen beladen. Im Apparat befinden sich verschiedene Träger, die sich durch ihre Partikelgröße oder Partikeldichte unterscheiden. Bedingt durch die unterschiedlichen physikalischen Eigenschaften der Träger wird erreicht, dass sich mehr Mikroorganismen anlagern können. Es bilden sich in vertikaler Richtung mehrere Bereiche der Wirbelschicht. Im unteren Bereich konzentrieren sich die großen oder schwereren Träger, darüber die feinen oder leichten. Somit wird auch das über der eigentlichen Wirbelschicht in Bodennähe liegende Apparatevolumen zur Anlagerung genutzt und somit die Verluste verringert.

Allgemeingültig kann festgestellt werden, dass Zellen von Mikroorganismen dadurch konserviert und geschützt werden, dass sie auf verschiedene Trägerstoffe schalenartig aufgesprüht werden oder dass bereits in einem Vorverarbeitungsschritt getrocknete Mikroorganismen unter Verwendung von zumeist wachs- oder fettartigen Bindemitteln agglomerieren.

Für derartige Prozesse werden häufig Wirbelschichtanlagen verwendet. Nachteilig ist bei diesen Prozessen, dass immer eine gewisse Mindestmenge an Partikeln im Wirbelschichtapparat enthalten sein muss, um das Binde- oder Coatingmittel durch Sprühdüsen aufzutragen. Diese minimale Füllhöhe ist abhängig von der Anordnung der Düsen und verhindert ein Durchsprühen der Düsen durch das Wirbelbett auf den Anströmboden. Diese Mindestmenge bestimmt im Falle eines kontinuierlich arbeitenden Prozesses die minimale Verweilzeit der Partikeln im Apparat. Dies ist nachteilig im Sinne der angestrebten Verweilzeitminimierung, da die Partikeln durch das Aufwirbeln mechanisch stark belastet sind und beispielsweise Coatingschichten zerstört werden können.

Für den Fall, dass in einem an sich bekannten Wirbelschichtapparat eine Flüssigkeit eingesprüht wird, die Kulturen enthalten, muss der Flüssigkeitsanteil zur Trocknung verdampft werden. Dazu ist Energie notwendig, die durch das erwärmte Fluidisierungsgas eingetragen wird. In direkter Nähe zu den Sprühdüsen werden die Partikeln mit der Flüssigkeit benetzt, und der Flüssigkeitsanteil verdampft von der Partikeloberfläche. Dies führt zu einer nicht vernachlässigbaren Temperaturungleichverteilung zwischen den unmittelbaren Eindüsungsorten und den äußeren Bereichen der Wirbelschicht. Die Partikelmenge ist dadurch im Wirbelschichtapparat immer einer thermischen Belastung ausgesetzt, da das Fluidisierungsgas an allen Bereichen des Anströmbodens mit einer gleich bleibenden Temperatur eintritt. Somit erfolgt außerhalb des Sprühbereiches eine ungewollte Erhitzung der Partikeln, was zu einer Schädigung der Mikroorganismen führt.

Aufgabe der Erfindung ist es, ein Verfahren zur Herstellung von Partikeln zu schaffen, bei dem die Teilchen kontinuierlich oder chargenweise unter Vermeidung von Temperaturungleichverteilungen hergestellt werden können. Dabei liegt das Anliegen darin, die Mikroorganismen direkt aus der Flüssigphase in eine gut rieselfähige Partikelform zu überführen.

Im Rahmen dieser Erfindung wird im weiteren Text der Begriff der flüssigen Formulierung verwendet um diejenige Flüssigkeit zu beschreiben, die mindestens eine Kultur von Mikroorganismen enthält. Weiterhin kann diese flüssige Formulierung Zusatzstoffe wie beispielsweise Bindemittel, Stabilisatoren, Nährstoffe usw. enthalten. In der Regel wird die flüssige Formulierung eine wässerige Lösung sein.
Die Zusammensetzung der Nährmedien für Mikroorganismen ist bekanntlich sehr unterschiedlich. Diese Nährmedien dienen grundsätzlich zur Vermehrung und zum Wachstum der Mikroorganismen, die industrielle Fertigung wird in Bioreaktoren vollzogen. Für Bakterien werden im allgemeinen Kohlenstoff und stickstoffhaltige Substanzgemische in wässeriger Lösungsform nach Sterilisation eingesetzt. Das Substrat für Pilze und Hefen enthält vorzugsweise Kohlenhydrate sowie Malzextrakt oder Melasse.
Das Verfahren des Züchtens der Mikroorganismen ist kein Bestandteil dieser Erfindung. Doch haben aus sehr unterschiedlichen Nährböden gewonnene Kulturen gemeinsam, dass diese im allgemeinen aus einem Gemisch von Flüssigkeit und Trockenstoff bestehen. Mehrheitlich handelt es sich hier um Wasser, andere organischen Lösemittel bleiben ausdrücklich vorbehalten.
Die Trockenstoffe, enthalten in der flüssigen Formulierung, sind von Natur her geeignet, um als Keimmaterial für den Granulataufbau zu dienen.

Der Erfindung erlaubt damit, mit und auch ohne zusätzliche Feststoffe den Trocknungs- und Granulierungsprozess im Gasstrahl zu vollziehen.
Zusätzliche Feststoffe im Gasstrahl werden im allgemeinen auf Grund deren Eigenschaften für den Metabolismus der lebendigen Mikroorganismen ausgewählt. Solche Feststoffe sind entweder von inerter Natur, wie Mineralstoffe, oder organischen Ursprungs, um als Nährboden zu dienen.
Auch können diese Feststoffe die von Mikroorganismen ausgeschiedenen Substanzen absorbieren, wie es z.B. der Fall ist mit homo-fermentativen Bazillen. Auch sind Feststoffe mit prebiotischer Wirkung wie Inulin und Oligofructose als Keimmaterial dienstlich.

Diese Aufgabe wird erfindungsgemäß durch die kennzeichnenden Merkmale des Patentanspruches 1 gelöst.

Erfindungsgemäß erfolgt die Herstellung von Partikeln durch Sprühgranulation durch eine Verknüpfung zwischen den thermischen Bedingungen in der Sprühzone und den Temperaturbedingungen im übrigen Bereich des Prozessraumes. Im erfindungsgemäßen Prozess wird dies dadurch erreicht, dass die Zuführung des erhitzten Prozessgases zur Trocknung ausschließlich im Bedüsungsbereich erfolgt. Die sichere Zuführung von Teilchen in den Bedüsungsbereich hinein erfolgt durch die spezielle geometrische Gestaltung des Apparates unter Nutzung der Schwerkraft.

Der Vorteil der erfindungsgemäßen Lösung besteht darin, dass die Herstellungsbedingungen an die herzustellenden Materialeigenschaften angepasst werden. Temperaturungleichverteilungen werden weitestgehend vermieden, wodurch auch eine Steigerung der Ausbeute und der Überlebensrate erreicht wird.

Weitere vorteilhafte Ausgestaltungen sind in den Unteransprüchen beschrieben, sie werden in der Beschreibung zusammen mit ihrer Wirkung erläutert.

Die Erfindung wird nachfolgend anhand eines Ausführungsbeispiels näher erläutert. In der dazugehörigen Zeichnungen ist schematisch eine Anlage zur Durchführung des erfindungsgemäßen Verfahrens dargestellt.
Die zur Trocknung der herzustellenden Granulate erforderliche Menge an erwärmtem Prozessgas 10 ( in der Regel erhitzte Luft oder auch inerte Gase ) wird einer Zuluftkammer 17, mit vorzugsweise rechteckigem Querschnitt 9 und begrenzenden Seitenwänden 5, zugeführt. In der Zuluftkammer 17 verteilt sich das Prozessgas 10 und tritt über Spaltöffnungen 1 in den Prozessraum 8 in Form von Gasstrahlen 2 ein. Der vorzugsweise horizontal in den Spalt 1 eintretende Prozessgasstrom wird durch das Umlenkteil 3 vorzugsweise nach oben in den Prozessraum 8 hinein umgelenkt und strömt als eine Art Freistrahl in den Apparat hinein. Im weiteren kann sich der Apparatequerschnitt optional in der Expansionszone 14 vergrößern, so dass sich die Geschwindigkeit der Prozessgasströmung nach oben hin stetig verringert. Das Gas verlässt den Apparat als Abgas 11 oberhalb der Expansionszone 14 über das Abluftteil 19, in das optional ein Entstaubungssystem (z.B. Filterpatronen oder Textilfilterelemente) integriert werden kann.

Im Prozessraum 8 befindet sich eine Menge an Partikeln, die durch den Prozessgasstrahl nach oben hin mitgerissen werden. Im oberen Bereich des Prozessraumes 8 sowie in der darüber befindlichen Expansionszone 14 nimmt die Gasgeschwindigkeit ab, so dass die aufwärts strömenden Teilchen seitlich aus dem Gasstrahl 23 heraustreten und in den Prozessraum 8 zurückfallen. Der Prozessraum 8 wird im unteren Bereich von geneigten Seitenwänden 29 begrenzt. Bedingt durch diese Seitenneigung werden die Teilchen unter Wirkung der Schwerkraft über die Rücklaufzone 24 in Richtung des Gaseintrittsspaltes 1 befördert, wo sie anschließend wieder vom Prozessgas in den Prozessraum 8 mitgerissen werden.

Durch diesen Mechanismus bildet sich eine sehr gleichförmige Feststoffzirkulation 15, bestehend aus einer Aufwärtsströmung und einem Rücklauf in Richtung des Prozessgaseintrittes. Dadurch liegt auch bei sehr geringen Mengen an Teilchen im Prozessraum 8 in der Kernzone oberhalb des Umlenkteiles 3 eine hohe Partikeldichte vor. In diesem Bereich werden ein oder mehrere Sprühdüsen 7 angeordnet, die gleichgerichtet zum Prozessgasstrahl nach oben sprühen und zum Einbringen der flüssigen Formulierung dienen.

Durch die hohe Partikelbeladung in der Kernzone ergeben sich in der Bedüsungszone 22 sehr vorteilhafte Bedingungen für den Wärme- und Stoffübergang. Weiterhin wird erreicht, dass sich die Flüssigkeit weitestgehend an den Teilchen abscheidet und diese somit gleichmäßig an den Partikeloberflächen benetzt werden. Das gleichmäßige Benetzen bei gleichzeitiger hohen Feststoffzirkulation zwischen Bedüsungsbereich und Rücklaufzone 24 bewirkt, das ein sehr gleichmäßiger Flüssigkeitsfilm gebildet wird. Durch den Trocknungsprozess verdampft die Flüssigkeit und verlässt mit dem Abgas 11 den Apparat. Der in der Formulierung enthaltene Feststoff verbleibt auf der Teilchenoberfläche. Dadurch wachsen die Granulate sehr gleichförmig und homogen, was auch ohne die Verwendung spezieller Klassiereinrichtungen ( z.B. Zick-Zack-Sichter ) zu einer sehr engen Korngrößenverteilung des Produktes führt.

Das Prozessgas kann einen Teil der Partikeln sowie Feingut und Stäube als feststoffbeladene Abluft 20 aus dem Prozessraum 8 ausgetragen. Zur Abscheidung dieser Teilchen kann das im Abluftteil 19 optional integrierte Filtersystem oder dem Apparat nachgeschaltete Entstaubungsanlagen verwendet werden. Im Falle einer integrierten Entstaubungsanlage 25 können beispielsweise Druckluftimpulse 18 genutzt werden, um die zurückgehaltenen Partikeln als abgetrennter Feststoff 21 in den Prozessraum 8 zurückzuführen.

Im Vergleich zu Wirbelschichtapparaten mit integrierten Filteranlagen wird die Staubrückführung dadurch erleichtert, dass die aufwärts gerichtete Prozessgasströmung im wesentlichen örtlich begrenzt ist und somit die zurückzuführenden Teilchen außerhalb des Gasstrahles sicher absinken können. Durch die Sogwirkung in der Nähe des Gaseintrittsspaltes 1 wird dieser Mechanismus zusätzlich gefördert. Alternativ können von der Abluft abgeschiedene Teilchen in den Prozessraum 8 zurückgeführt werden. Dazu können im unteren Bereich der geneigten Seitenwände 29 verschiedenartigste Zuführungen 26 angeordnet sein. Bedingt durch die hohe Geschwindigkeit des Prozessgasstrahls in der Nähe des Gaseintrittsspaltes 1 werden die feinen Partikeln angesaugt und der Bedüsungszone 22 zugeführt, wo diese mit Flüssigkeit benetzt werden und am Wachstumsprozess teilnehmen.

Optional eingebaute Leitbleche 16 stützen den Gasstrahl, verstärken den Sogeffekt und verbessern die Zuführung der Feststoffe in der Bedüsungszone 22 hinein. Eventuell auftretende Agglomerationseffekte werden minimiert, da im Bedüsungsbereich sehr hohe Strömungsgeschwindigkeiten und somit höhere Trennkräfte als in Wirbelschichten auftreten. Dadurch werden Teilchen separiert und wachsen zu sehr kugeligen Granulaten.

Das Strömungsprofil des Prozessgases im Prozessraum 8 bewirkt weiterhin, dass von der optional integrierten Filteranlage in den Prozessraum zurückgeführte Feinpartikel nicht in die Bedüsungszone 22 zurückfallen. Dadurch wird das Verkleben von Feinpartikeln und daraus folgende Agglomeratbildungsprozesse unterbunden.

Zur kontinuierlichen Prozessführung kann der Apparat mit optional unterschiedlichen Eintragsystemen 13 für Feststoffe ausgerüstet werden. Dadurch können beispielsweise Partikel dem Prozess zugeführt werden, die durch Zerkleinerung von beispielsweise (zu großen) Granulaten gewonnen werden können oder/und aus zu kleinen Granulaten bestehen. Diese Teilchen dienen dann als Granulationskeime oder als Startfüllung zur Verkürzung der Inbetriebnahmezeit. Außerdem können hier Additive, Trägerstoffe oder andere Hilfsstoffe in fester Form in den Prozess eingeschleust werden, die in die Granulate eingebettet werden sollen.

Weiterhin kann der Apparat mit Austragsorganen 4 versehen werden, um Partikel aus dem Prozessraum 8 entnehmen zu können. Dies kann beispielsweise durch einen Überlauf oder durch ein volumetrisches Austragsorgan (z.B. eine Zellenradschleuse) oder auch durch einen Schwerkraftsicher (z.B. einen mit Sichtgas beaufschlagten Zick-Zack-Sichter oder einen Steigrohrsichter) erfolgen.

Optional können mechanische Aggregate 27 im Prozessraum 8, jedoch vorzugsweise im Bereich der Rücklaufzone 24 an den geneigten Wänden angebracht werden, um durch Zerkleinerung ausreichend Feinmaterial als Keime für den Granulatbildungsprozess zu erzeugen. Weiterhin kann die Rücklaufzone 24 optional zur Positionierung von Beheizungen oder anderen Wärmeübertragungseinrichtungen 28 genutzt werden. Beispielsweise kann die Apparatewand doppelwandig ausgeführt sein, um diese beispielsweise unter Nutzung von flüssigen oder gasförmigen Wärmeträgern zur Beheizung oder Kühlung zu verwenden. Alternativ könnten auch Mikrowellenheizer genutzt werden, um die Partikel in der Rücklaufzone 24 nachzutrocknen oder vorzuwärmen.

Im Prozessraum 8 oder in den darüberliegenden Apparateteilen, der Expansionszone 14 und dem Abluftteil 19, können optional Sprühdüsen 6 angeordnet werden, die vorzugsweise nach unten aber auch teilweise nach oben sprühen. Hier kann ebenfalls die flüssige Formulierung eingedüst werden, um beispielsweise durch Sprühtrocknung im Apparat Granulationskeime zu erzeugen. Alternativ können über einige der Sprüheinrichtungen 6 und 7 Additive oder andere Komponenten in flüssiger Form eingesprüht und somit in die Granulatstruktur homogen eingebettet werden. Wenn die Sprühdüsen 7 die heißgasbeaufschlagte Zuluftkammer 17 passieren, können optional die flüssigkeitsführenden Teile mit Isolationen oder unterschiedlichen Kühlsystemen 12 versehen werden, um Schädigungen an der flüssigen Formulierung zu unterbinden.

Zum Schutz vor beispielsweise Luftsauerstoff und Feuchtigkeit sowie zur Verbesserung der Lagerstabilität können die erzeugten Partikel in einem nachfolgenden separaten Prozess oder im gleichen Apparat durch Coating mit einem Schutzüberzug versehen werden. Mögliche Coatingmaterialien können sein Fette oder Wachse, Shellac oder Polymere der Familien Methacrylsäure oder Methacrylat.
Als weiterer Vorteil des erfindungsgemäßen Prozesses ist der sehr einfache Aufbau zu nennen, der eine hohe Betriebssicherheit und Störungsunempfindlichkeit mit sehr guter Reinigbarkeit verbindet. Somit werden verbesserte Produktionsbedingungen insbesondere hinsichtlich der Hygieneanforderungen bei Produktwechsel bei biologischen Stoffen geschaffen.

### Beispiele:

Die Erfindung wird anhand von Anwendungsbeispielen veranschaulicht, ohne dadurch in irgend einer Weise eingeschränkt zu werden.

### Beispiel 1: Verkapselung und Immobilisierung von Bifidus bifidum

Bazillen der Gattung Bifidus bifidum wurden inokuliert in einem Nährboden bestehend aus de Man, Rogosa, Sharpe Bouillon von 100 g in einer Lösung auf 1,92 Liter destilliertem Wasser. Die Zellkonzentration der Formulierung wurde bis auf 14·10⁶ Kbe/g vermehrt und anschließend im Verfahren gemäß der Erfindung unter Zugabe von 300 g Maltodextrin mit einer Luftmenge von ca. 125 m³/h und 60 °C bis < 5 % Wassergehalt getrocknet und granuliert.
Der Anzahl der Kolonien nach Ablauf des Verfahrens gemäß der Erfindung hatte sich vermehrt auf 18·10⁷ Kbe/g (Kbe = Kolonie bildende Einheiten).

### Beispiel 2: Verkapselung und Immobilisierung von Bifidus bifidum

Bazillen der Gattung Bifidus bifidum wurden inokuliert in einem Nährboden bestehend aus de Man, Rogosa, Sharpe Bouillon von 100 g in einer Lösung auf 1,92 Liter destilliertem Wasser. Milchsäure wurde präzipitiert durch Zugabe von Calciumcarbonat, dieses wurde wiederholt bis zum Erreichen von einer Zelllkonzentration von 17·10⁹ Kbe/g in der Biomasse. Die Formulierung wurde im Verfahren gemäß der Erfindung unter Zugabe von 300 g Maltodextrin mit einer Luftmenge von ca. 125 m³/h und 60 °C bis < 5 % Wassergehalt getrocknet und granuliert.
Der Anzahl der Kolonien nach Ablauf des Verfahrens gemäß der Erfindung wurde mit 18·10⁹ Kbe/g gemessen.

### Beispiel 3: Verkapselung und Immobilisierung von Bifidus bifidum

Bazillen der Gattung Bifidus bifidum wurden inokuliert in einem Nährboden bestehend aus de Man, Rogosa, Sharpe Bouillon von 50 g in einer Lösung auf 1,92 Liter destilliertem Wasser, bis zum Erreichen eines pH-Wertes von 5.0. Eine Mischung von gefriergetrockneten Bifidus bifidum - Zellen und Molkepulver wurde bis zum Erreichen von einem Zellbestand von 16·10⁹ zugegeben.
Die Formulierung wurde ohne Beigabe von Kernmaterial mit einer Luftmenge von ca. 125 m³/h und 60 °C bis < 5 % Wassergehalt getrocknet und granuliert.
Der Anzahl der Kolonien nach Ablauf des Verfahrens gemäß der Erfindung wurde mit 16·10⁹ Kbe/g gemessen.

### Beispiel 4: Verkapselung und Immobilisierung von Bifidus bifidum

Bazillen der Gattung Bifidus bifidum wurden inokuliert in einem Nährboden bestehend aus de Man, Rogosa, Sharpe Bouillon von 50 g in einer Lösung auf 1,92 Liter destilliertem Wasser, bis zum Erreichen eines pH-Wertes von 5.0. Eine Mischung von gefriergetrocknete Bifidus bifidum - Zellen und Molkepulver wurde bis zum Erreichen von einem Zellbestand von 16·10⁹ zugegeben.
Die Formulierung wurde mit Beigabe von 100 g Maltodextrin als Kernmaterial mit einer Luftmenge von ca. 125 m³/h und 60 °C bis < 5 % Wassergehalt getrocknet und granuliert.
Durch die gleiche Düsenvorrichtung wurde 100 g einer Schmelze bestehend aus Mono-Diglycerid verdüst und das Granulat mit einer Hülle von ca. 20 µm verkapselt.
Der Anzahl der Kolonien nach Ablauf des Verfahrens gemäß der Erfindung wurde mit 22·10⁹ Kbe/g gemessen.

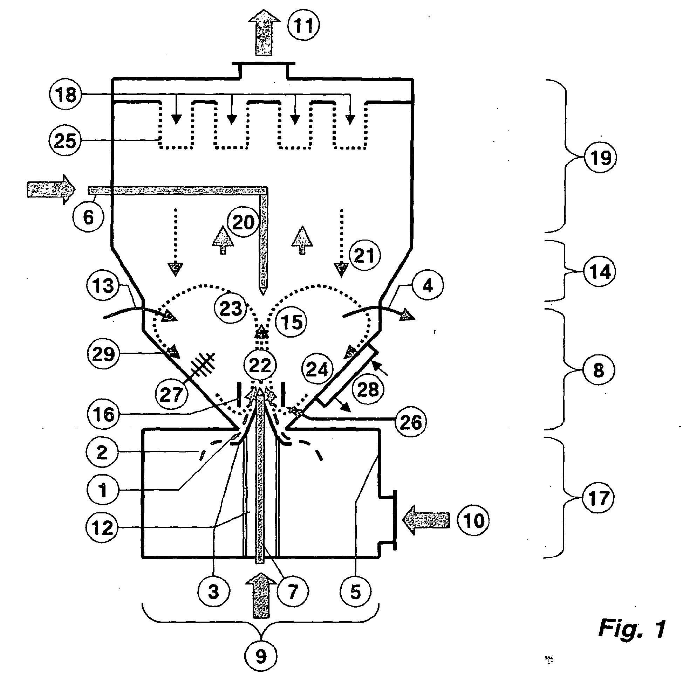

## Patentansprüche

1. Verfahren zur Herstellung von Granulaten,
**dadurch gekennzeichnet, dass**
a.ein oder mehrere flüssige Formulierungen über Sprüheinrichtungen in einen feststoffbeladenen Gasstrahl eingedüst werden,
b.die flüssige Formulierung mindestens eine Kultur von Mikroorganismen enthält
c.die mit Flüssigkeit benetzten Materialteilchen im erwärmten Gasstrahl einem Trocknungs- und Granulationsprozess unterzogen werden,
d. die Teilchen nach einer Verweilzeit vom Gasstrahl getrennt und in den Prozessraum zurückgeführt werden,
e.die Teilchen durch Schwerkraft über geneigte Flächen dem Gaseintrittsbereich zugeführt werden,
f.Feinpartikel, Stäube und vom Prozessgas mitgerissene Teilchen abgeschieden werden und dem Prozess als Keimmaterial für den Granulatbildungsprozess wieder zugeführt werden,
g. durch Materialzuführung in den über die vorzugsweise rotationssymmetrische oder langgestreckte Spaltöffnungen zugeführten Gasstrahl(en) eine in axialer Richtung des Reaktionsraumes liegende kreisähnliche Feststoffströmung erzeugt wird,
h. der Granulationsprozess kontinuierlich oder chargenweise durchgeführt wird.

2. Verfahren nach Anspruch 1, **dadurch gekennzeichnet, dass** die Granulate über unterschiedliche Sichtvorrichtungen aus dem Prozessraum entnommen werden.

3. Verfahren nach Anspruch 1, **dadurch gekennzeichnet, dass** die Granulate über unterschiedliche volumetrische Austragsorgane aus dem Prozessraum entnommen werden.

4. Verfahren nach einem oder mehren Ansprüchen, **dadurch gekennzeichnet, dass** zu große oder zu kleine aus dem Prozess entnommene Granulate vom Gutprodukt abgetrennt werden.

5. Verfahren nach einem oder mehren Ansprüchen, **dadurch gekennzeichnet, dass** zu kleine aus dem Prozess entnommenen Granulate in den Prozessraum als Keimmaterial zurückgeführt werden.

6. Verfahren nach einem oder mehren Ansprüchen, **dadurch gekennzeichnet, dass** zu große aus dem Prozess entnommenen Granulate durch ein beliebiges Zerkleinerungsaggregat zerkleinert und in den Prozessraum als Keimmaterial zurückgeführt werden.

7. Verfahren nach einem oder mehren Ansprüchen, **dadurch gekennzeichnet, dass** die in den Prozessraum zurückgeführten Granulate thermisch nachbehandelt werden.

8. Verfahren nach Anspruch 7, **dadurch gekennzeichnet, dass** die in den Prozessraum zurückgeführten Granulate getrocknet oder vorgewärmt werden.

9. Verfahren nach einem oder mehren Ansprüchen, **dadurch gekennzeichnet, dass** die in den Prozessraum zurückgeführten Granulate zerkleinert werden.

10. Verfahren nach einem oder mehren Ansprüchen, **dadurch gekennzeichnet, dass** Granulate aus unterschiedlichen Zusätzen und mit unterschiedlichen Mischungsverhältnissen hergestellt werden.

11. Verfahren nach einem oder mehren Ansprüchen, **dadurch gekennzeichnet, dass** dem Prozess zusätzliche Hilfsstoffe in fester Form ( Pulver, Kristalle, o.ä.) zugeführt werden.

12. Verfahren nach einem oder mehren Ansprüchen, **dadurch gekennzeichnet, dass** die erzeugten Granulate im gleichen Apparat oder anschließend- ein oder mehrschichtig gecoatet werden.

13. Verfahren nach einem oder mehren Ansprüchen, **dadurch gekennzeichnet, dass** das Coatingmaterial ein Fett oder Wachs ist.

14. Verfahren nach einem oder mehren Ansprüchen, **dadurch gekennzeichnet, dass** das Coatingmaterial Shellac ist.

15. Verfahren nach einem oder mehren Ansprüchen, **dadurch gekennzeichnet, dass** das Coatingmaterial ein Polymer der Familien Methacrylsäure oder Methacrylat ist.

## Claims

1. Method of preparing granulates. **characterised in that**
a. one or more liquid formulations are sprayed by means of spray devices into a solids-charged jet of gas,
b. the liquid formulation contains at least one culture of microorganisms,
c. the particles of material wetted with liquid are subjected to a drying and granulation process in the heated gas jet,
d. after a dwell time the particles are separated from the gas jet and recycled into the processing chamber.
e. the particles are gravity-fed over inclined surfaces into the gas inlet area,
f. fine particles, dust and particles picked up by the processing gas are separated off and fed back into the process as nuclei material for the granulation process,
g. a flow of solids resembling a circle and situated in the axial direction of the reaction chamber is produced by feeding material into the gas jet(s) passing through slit-like openings which are preferably rotationally symmetrical or elongate,
h. the granulation process is carried out continuously or batchwise.

2. Method according to claim 1, **characterised in that** the granules are removed from the processing chamber through various sifting devices.

3. Method according to claim 1, **characterised in that** the granules are removed from the processing chamber through various volumetric discharging means.

4. Method according to one or more claims, **characterised in that** oversized or undersized granulates removed from the process are separated from the satisfactory product.

5. Method according to one or more claims, **characterised in that** undersized granulates removed from the process are fed back into the processing chamber as nuclei material.

6. Method according to one or more claims, **characterised in that** oversized granulates removed from the process are comminuted using any desired comminution equipment and fed back into the processing chamber as nuclei material.

7. Method according to one or more claims, **characterised in that** the granulates fed back into the processing chamber are subjected to a thermal after-treatment.

8. Method according to claim 7, **characterised in that** the granulates fed back into the processing chamber are dried or preheated.

9. Method according to one or more claims, **characterised in that** the granulates fed back into the processing chamber are comminuted.

10. Method according to one or more claims. **characterised in that** granulates are prepared from different added batches and in different mixing ratios.

11. Method according to one or more claims. **characterised in that** additional adjuvants in solid form (powders. crystals, or the like) are fed into the process.

12. Method according to one or more claims, **characterised in that** the granulates produced are coated with one or more layers in the same apparatus or subsequently.

13. Method according to one or more claims, **characterised in that** the coating material is a fat or wax.

14. Method according to one or more claims, **characterised in that** the coating material is shellac.

15. Method according to one or more claims. **characterised in that** the coating material is a polymer from the methacrylic acid or methacrylate families.

## Revendications

1. Procédé pour la fabrication de granulés,
**caractérisé en ce que**
a. une ou plusieurs formulations liquides sont injectées dans un flux de gaz chargé en matières solides par l'intermédiaire de dispositifs de pulvérisation,
b. la formulation liquide contient au moins une culture de microorganismes
c. les particules solides humidifiées avec le liquide dans le flux de gaz chauffé sont soumises à un processus de séchage et de granulation,
d. les particules sont séparées du flux de gaz après une certaine durée et sont reconduites dans la chambre de traitement,
e. les particules sont acheminées jusqu'à la zone d'entrée du gaz par l'effet de la gravité par l'intermédiaire de surfaces inclinées,
f. des particules fines, poussières et particules entraînées par le gaz de traitement sont éliminées et sont ajoutées au processus sous la forme d'un matériau de germination pour le processus de formation de granulé,
g. un flux de matière solide presque circulaire se trouvant dans la direction axiale de la chambre de réaction est produit grâce à l'apport en matériau dans le(s) flux de gaz passant dans les ouvertures à fentes de préférence allongées ou à symétrie de révolution,
h. le processus de granulation est réalisé en continu ou par charges.

2. Procédé selon la revendication 1, **caractérisé en ce que** les granulés sont extraits de la chambre de traitement par l'intermédiaire de différents dispositifs de séparation.

3. Procédé selon la revendication 1, **caractérisé en ce que** les granulés sont extraits de la chambre de traitement par l'intermédiaire de différents organes de sortie volumétriques.

4. Procédé selon une ou plusieurs des revendications précédentes, **caractérisé en ce que** des granulés trop gros ou trop petits extraits du processus sont séparés du produit de qualité.

5. Procédé selon une ou plusieurs des revendications précédentes, **caractérisé en ce que** des granulés trop petits extraits du processus sont reconduits dans la chambre de traitement sous la forme de matériau de germination.

6. Procédé selon une ou plusieurs des revendications précédentes, **caractérisé en ce que** des granulés trop gros extraits du processus sont broyés par un agrégat de broyage souhaité et reconduits dans la chambre de traitement sous la forme de matériau de germination.

7. Procédé selon une ou plusieurs des revendications précédentes, **caractérisé en ce que** les granulés reconduits dans la chambre de traitement sont retraités de façon thermique.

8. Procédé selon la revendication 7, **caractérisé en ce que** les granulés reconduits dans la chambre de traitement sont séchés ou préchauffés.

9. Procédé selon une ou plusieurs des revendications précédentes, **caractérisé en ce que** les granulés reconduits dans la chambre de traitement sont broyés.

10. Procédé selon une ou plusieurs des revendications précédentes, **caractérisé en ce que** l'on fabrique des granulés à partir de divers additifs et selon des proportions de mélange différentes.

11. Procédé selon une ou plusieurs des revendications précédentes, **caractérisé en ce que** des adjuvants supplémentaires sont ajoutés sous une forme solide (poudre, cristaux, etc.) au processus.

12. Procédé selon une ou plusieurs des revendications précédentes, **caractérisé en ce que** les granulés produits sont enrobés d'une ou plusieurs couches dans le même appareil ou bien par la suite.

13. Procédé selon une ou plusieurs des revendications précédentes, **caractérisé en ce que** le matériau d'enrobage est une graisse ou une cire.

14. Procédé selon une ou plusieurs des revendications précédentes, **caractérisé en ce que** le matériau d'enrobage est du Shellac.

15. Procédé selon une ou plusieurs des revendications précédentes, **caractérisé en ce que** le matériau d'enrobage est un polymère de la famille de l'acide de méthacrylate ou du méthacrylate.
